# EUROPEAN PATENT APPLICATION

(11) **EP 1 970 069 A1**
(43) Date of publication of application: **17.09.2008**
(21) Application number: 07005468.9
(22) Date of filing: 16.03.2007
(51) Int. Cl.: A61K 36/53, A61P 31/00

(54) **Medicament containing Danshen for use in prevention and/or treatment of infections**

(71) Applicant: Nutri-Fit GmbH & Co. KG, 49439 Mühlen (DE)
(72) Inventor: Schemmer, Peter Prof. Dr., 69181 Leimen (DE); Sobirey, Michael Dr., 26160 Bad Zwischenahn (DE); Schneider, Heinz Dr., 1792 Cordast (CH)
(74) Representative: Bösl, Raphael Konrad

(57) **Abstract**

The present invention relates to a medicament comprising Danshen for use in prevention and/or treatment of an infection following surgical intervention and the use of Danshen for the manufacture of a medicament for prevention and/or treatment of an infection following surgical intervention and optionally a post-operative non-infectious complication.

## Description

The present invention relates to a medicament comprising Danshen for use in prevention and/or treatment of an infection following surgical intervention and the use of Danshen for the manufacture of a medicament for prevention and/or treatment of an infection following surgical intervention and optionally a post-operative non-infectious complication.

Despite improved technical handling of surgical cases, postoperative infectious and non-infectious complications represent a significant source of morbidity and mortality and related medical treatment costs continue to be a major burden for any health care system. Disruption of the microvasculature is a key factor in the mechanism of e.g. hepatic, mesenteric and cardiac ischemia-reperfusion injury occuring during major operative procedures. Furthermore, surgery-induced alterations of the immune and inflammatory system are well described and may play a role in the genesis of postoperative complications. It has been recognized that an exaggerated inflammatory response and with it important injury (oxidative tissue damage, also in distant organs, i.e. lungs; microvascular leakage; neutrophil-endothelium adhesion) results from tissue ischemia and the following reperfusion, particularly in visceral tissues. This exaggerated inflammatory response together with blood transfusions and/or hemorrhage contribute to a suppression of the immune system.

In order to prevent infectious postoperative infections pre- and perioperative application of antibiotics has become standard procedure in surgery. However, the efficacy of a single administration of antibiotics immediately before or during surgical treatment is till being discussed. Postoperative complications do occur in spite of preventive treatment with antibiotics immediately before or during surgical treatment and these have so far been treated upon their occurrence during the postoperative recovery period.

However, there exist some descriptions of approaches according to which a patient may be prepared before a planned surgical procedure by preoperative application of specific nutritional formulations in order to reduce the risk and the severity of postoperative complications.

In the patent US-A-5,656,608 a method is described for the treatment of endotoxemia by application of a therapeutically active quantity of the amino acids glycine, alanine and serine for at least a period of three days prior to surgery. In the patent US-A-5,731,290 a method is disclosed to improve the immune response after surgical procedures via preoperative application of a food supplement containing an immune-stimulating quantity of omega-3 fatty acids combined with L-arginine, L-omithine or their precursors. The supplement may be given for at least three days before surgery. In the patent US-A-5,902,829 a method is disclosed to modulate microcirculation in a patient undergoing elective or emergency surgery by preoperatively administering an NO-donor such as L-arginine and its precursors. The administration is initiated at least one day prior to surgery. WO-A-96/25,861 describes the use of glycine and glycine precursors, alanine and serine for preparing a medicament or a nutritional formulation to reduce endotoxemia in patients undergoing surgical procedures. Preopeative administration is required for a period of at least 3 days before surgery. From WO-A-99/62,508 it is known that glycine can be used to formulate a medication for the treatment of hemorrhagic shock describing in addition the preoperative use of such a formulation. US-A-6,013,273 describes a method to treat endotoxic shock by the use of an appropriate amount of choline, which is administrated over a period of at least one day perioperatively, but normally during a period of one to six days before surgery. Furthermore, the patent application WO 2005/102319 describes the use of melatonin in preventing postoperative infectious complications such as pneumonia, wound infection, intra-abdominal abscess (peritonitis), urinary tract infections (UTI) and noninfectious complications such as anastomotic leak.

Common denominator of all of the above mentioned treatments is the fact that all of them have to be initiated at least one or more days before the actual surgical procedure is carried out. However, such approaches are not feasible for treating an emergency, i.e. a trauma patient requiring an immediate operative procedure not allowing a pretreatment time of a day or more. Additionally, the results achieved by the above methods have not been satisfying. Finally, it is generally accepted that administration of antibiotics stresses the _patient's organism and frequent use of antibiotics results in the development of antibiotic resistance.

Therefore, there is still a need for alternative medicament in order to treat or prevent an infection, particular a post-operative infection. Preferably, the alternative medicament provides for a prophylatic treatment, more preferably wherein the medicament allows short term preoperative treatment of a patient - e.g. within a few hours to a few minutes - for the purpose of preventing or prophylactically or therapeutically treating postoperative infectious and optionally non-infectious complications induced by major surgical.

Surprisingly, it was now found that a medicament comprising Danshen may be useful in the prevention and/or treatment of a postoperative infection. Therefore, the use of Danshen may reduce the length of stay in hospital of a patient who undergoes a surgical intervention. Therewith the average treatment costs of patients undergoing operative procedures may be reduced.

Major surgery is a powerful stimulus that provokes a systemic inflammatory response which can be identified as a relevant risk factor which may lead to a state of hypermetabolism, immunological dysfunction and organ deterioration. Some of the currently accepted underlying mechanisms for the massive inflammatory response occuring during major surgery include bacterial translocation and systemic endotoxemia followed by an excessive activation of the immune system (acute phase response) mediated through TNF-α and interleukins (IL-6, IL-8) and a failure to maintain antioxidant defences (oxidative stress). Animal models of surgery and patients undergoing major operative procedures exhibit oxidative stress characterized by elevated free radical generation that may be causative in the local wound response and in the development of infectious complications as well as distant organ injury (failure). The oxidative stress-induced suppression of nonspecific resistance and the disturbance in the adaptive immune system makes surgical patients vulnerable to infections. Danshen contains powerful antioxidative compounds and is a potent protective agent against free radical induced cell damage after experimental surgical injury (as shown by our examples). Without being bound to this threory, inventors assume that some actions of Danshen as a potential supportive pharmacologic agent in surgical patients include its role as a scavenger of both oxygen and nitrogen-based reactants, stimulation of the activities of a variety of antioxidative enzymes and/or reduction in proinflammatory cytokines. These combined actions of Danshen, along with its low toxicity, could make it a ubiquitously acting and highly beneficial agent in preventing infectious complications following major surgery.

Accordingly, a first subject of the invention relates to a medicament comprising Danshen for use in the prevention and/or treatment of an infection following surgical intervention.

Danshen represents the dried roots (rhizomes) of *Salvia miltiorrhiza.* In traditional Chinese medicine, the medicinal product Danshen (also referred to as Ch'ih Shen (scarlet sage), DanShen, Dan Shen, danshen root, Ten Shen, Huang Ken, Hung Ken (red roots), Pin-Ma Ts'ao (horse-racing grass), *Radix salvia miltiorrhiza,* red-rooted sage, red sage root, red saye root, *Salvia bowelyana, Salvia miltiozzhiza bunze, Salviae miltiorrhizae, Salvia przewalskii, Salvia przewalskii mandarinorum, Salvia yunnanensis,* salvia root, Sh'ih Shen, Shu-Wei Ts'ao (rat-tail grass), Tan Seng, Tan-Shen, Tzu Tan-Ken (roots of purple sage), Chinese Sage, Huang Ken, Radix Salvia or Salvia Root) is widely used, often in combination with other herbs, to treat heart conditions and strokes. Danshen has been approved by the Chinese National Drug Administration as standard treatment for diseases associated with an ischemic process such a myocardial infarction, cerebral infarction, occlusive vasculitis and atherosclerosis. Results from animal and human studies support these uses of Danshen. Danshen is available by a series of commercial suppliers, particularly in China, and can be purchased e.g. at Shanghai Tongyoung Medical Co. Ltd., Z20027936, Nr. 030214, People's Republic of China.

The main active constituents of *Salvia miltiorrhiza* are diterpene quinones, known as tanshinones. Most of these compounds are colored, providing the reddish appearance of the roots. The group of components, referred to as tanshinone I, tanshinone II and cryptotanshinone were first described by researchers in 1968, though investigations had been underway since the pigments were isolated from *Salvia miltiorrhiza* in 1934. More recently, nearly 40 variants of the basic tanshinone structures have been found in the roots. The tanshinones are unique chemical constituents, and similar compounds are not found in other Chinese herbs. The total tanshinone content of the roots is about 1 %, with tanshinone I and II and cryptotanshinone being present in the largest amount.

Danshen is widely known to decrease the blood's ability to clot in at least two ways. First, it limits the stickiness of blood components known as platelets. Secondly, it also decreases the production of fibrin threads of protein that trap blood cells to form clots. These effects help to improve central as well as peripheral blood circulation.

Further beneficial effects of Danshen have been reported. Particularly, it has been reported that the administration of infusion of Danshen extract solution to patients undergoing hepatectomy before vascular occlusion prevents hepatocytes from reperfusion injury (Chinese Journal Hua Xi Yi Ke Da Xue Xue Bao. 1997; 28(2):197-200), that the intravenous administration of Salvia miltiorrhiza composita (SMC) during the operation on patients with ischemic coronary heart disease (CHD) undergoing non-heart surgery could effectively improve and protect myocardial ischemia while no side effects were observed (Chinese Journal Zhongguo Zhong Xi Yi Jie He Za Zhi. 1999; 19(2):75-6), that Composite Salviae Injection (CSI) showed a gastrointestinal protective effect of in patients undergoing cardiopulmonary bypass heart surgery (Chinese Journal Zhongguo Zhong Xi Yi Jie He Za Zhi. 2001; 21(3), 177-9) Salvia miltiorrhiza injection after anesthesia induction and at the time of rewarming reduced myocardial damage (creatine kinase-MB) and attenuated postoperative vasoactive mediator imbalance (J Thorac Cardiovasc Surg. 2003 Nov;126(5):1404-10), that Danshen injection on early stage after renal transplantation helps to improve blood microcirculation and decreases the incidence of retardation of renal function recovery (Chinese Journal Zhongguo Zhong Xi Yi Jie He Za Zhi. 2005; 25(5):404-7) and that pretreatment with Salvia miltiorrhiza (250 mg/kg) before induction of hepatic I/R injury could partially restore intestinal microflora balance, improve intestinal mucosal integrity, and reduce bacterial translocation and plasma endotoxin in rats (Hepatobiliary Pancreat Dis Int. 2005; 4(2):274-80).

However, none of these publications relates to or indicates the effects of Danshen on postoperative infections.

An "infection following surgical intervention" or "postoperative infection" relates to any infectious complication following surgical procedures. Particularly, the infection occurs between 3 to 9 days after actual operation, especially between 4 to 7 days after actual operation. The complication may be any unfavorable evolution of a disease, a health condition or a medical treatment following the surgical intervention and being caused by an infection. An infection is the detrimental colonization of a host organism, i.e. the patient who underwent surgical intervention, by a foreign species. Such infections are observed with relative frequency, even after "sterile" operations, particularly in a time window of 3 to 9 days, especially 4 to 7 days following the operative procedure. An estimated 5 to 10 percent of hospitalized patients undergoing otolaryngology surgery acquire a nosocomial infection, which adds a substantial cost and an average of 4 extra days to the hospital stay. Urinary tract infections are the most common nosocomial infections (accounting for more than 7 million physician visits every year in the United States), and are second in seriousness to respiratory infections.

In a preferred embodiment the medicament is used in the prevention of an infection following surgical intervention. In accordance with this embodiment, the medicament is to be administered to the subject before appearance of any symptoms of an infection such as fever, fatigue/tiredness, tumescence, redness, pain and/or nausea.

In an even more preferred embodiment of the invention the medicament is to be administered prior to the surgical intervention. Prior to the surgical intervention with in the context of the present invention means that the medicament is to be administered before the surgical intervention is started, i.e. before the first incision into the patient's body.

Still more preferably, the medicament is to be administered at most approximately 24 hours prior to the surgical intervention, preferably at most approximately 12 hours prior to the surgical intervention, more preferably at most approximately 6 hours prior to the surgical intervention, even more preferably at most approximately 3 hours prior to the surgical intervention, still more preferably at most approximately 2 hours prior to the surgical intervention, most preferably at most approximately 1 hour prior to the surgical intervention.

In another preferred embodiment of the invention, the medicament according to the invention is to be administered at most three times, preferably at most twice or once before or during the surgical intervention in order to prevent an infection following surgical intervention. If the surgical intervention does not take last for a long time, e.g. 1 or 2 hours, a single administration of the medicament of the invention might be sufficient in order to prevent the infection. However, if the surgical intervention lasts for several hours, such as 4 to 6 hours or even longer, it might be necessary to administer a second or even third dose of the medicament of the invention in order to maintain the protective effect of the medicament.

However, the finding that the use of Danshen at most approximately 3 to 1 hour prior to the surgical intervention has the above advantages; this use is particularly suited for application in case of an emergency, i.e. where the surgical intervention was not planned. Depending on the route of administration the period of time between the administration and the surgical intervention can even be shorter. Particularly, when Danshen is administered into the circulation (e.g. intra-arterially or intravenously) and immediately distributed in the body of the patient to be operated, the period of time can be as short as at most 30 min, preferably at most 20 min, more preferably, at most 15 min, still more preferably at most 10 min. In the context of the present invention, the period of time between the administration and the surgical intervention is the time between the patient's uptake of the medicament and the first incision into the patient's body by the physician, dentist, or veterinarian.

In a preferred embodiment of the invention the medicament is to be administered in a daily dose of from 0.1 to 5000 mg/kg body weight, preferably 1 to 2000 mg/kg body weight, more preferably 5 to 1000 mg/kg body weight and most preferably 50 to 500 mg/kg body weight. The dose of 1 to 5000 mg/kg body weight corresponds to a daily dose of about 50 mg to 500g Danshen (body weight range of from 50 to 100 kg). However, the skilled practitioner will understand that the amount of Danshen to be administered to a subject will depend to a large extent to the patients' specific requirements relying on a series of factors (such as the age, sex, weight and condition of the patient, the surgical intervention intended, a possible interaction with other drugs to be administered etc., the duration and frequency of administration of Danshen, the infection to be treated or preferably prevented (which is either already known or preferably which is most probable to occur) and it will be within the skills of the attending physicians to determine a suitable effective amount of Danshen for administration to the patient.

In accordance with the prophylactic treatment (i.e. prevention), the administration of Danshen may be started before the surgical intervention and it may or may not be continued during or after the surgical intervention, e.g. for up to about 14 or about 10 days, such as 9, 8, 7, 6, 5, 4, 3 or 1 day(s). For example, the formulation of the invention may be intravenously or intra-arterially administered as single-shot application or as single-shot application in combination with another agent such as an antibiotic (e.g. 1.5 g of third generation cephalosporins) that are routinely used for infectious prophylaxis. A second dose of Danshen or Danshen combined with e.g. an antibiotic may be given if the duration of the operation is longer than four hours or with an intraoperative blood loss of at least one 1 liter. Further doses of Danshen may be administered during the post-operative course.

The medicament may be in any suitable form. For use in the within the present invention Danshen is conveniently employed in dried (powdered) form as described in Pharmacopoeia of the People's Republic of China (English Edition 2000, Vol.1, pages 192-193), optionally pressed into tablets, or in the form of concentrated solutions. The medicament may be administered to the patient orally, enterally, transdermally or parenterally. The galenic formulation of the medicament may be chosen in accordance with the intended route of administration and may be prepared in a manner known per se, e.g. by admixing the ingredients.

However, in a preferred embodiment the medicament is in a form intended for oral administration, preferably a drinking solution, decoction, tablet, a hard gelatin capsule, a soft gelatin capsule or in a form intended for parenteral administration, preferably for intravenous or intra-arterial administration. Furthermore, the medicament may be administered into the stomach of the patients via gastric tube following intubation for general anesthesia.

If the medicament is intended for the intravenous or intra-arterial application, a parenteral formulation is preferred. Typical pharmacologically acceptable formulation forms for intravenous or intra-arterial administration will further comprise pharmacologically acceptable diluents, carriers, microemulsions, pigments and/or other adjuvants well known to the skilled person to be suitable for incorporation into such formulation and optionally another agent such as an antibiotic suitable for preoperative phrophylaxis (see below).

The medicament according to the present invention may be used as the sole therapeutically active agent or it may be used in combination with another infection prevention and/or treatment agent, preferably in combination with an antibiotic. "As a sole therapeutically active agent" with in the present invention means that Danshen is used as any of the typical formulations of the extract of *Salvia miltiorrhiza* (e.g. those described above) without any further therapeutically active agent or ingredient. However, Danshen may also be combined with another agent against an infection. When combined both agents (Danshen and the other infection prevention and/or treatment agent) may be used simultaneously and/or consecutively. If used simultaneously, both agents may be combined into one formulation or may be administered as individual formulations. Preferably, Danshen is combined with an antibiotic. Examples of suitable antibiotics include without limitation those selected from the any of the following classes: aminoglycosides (such as amikacin, gentamicin or tobramycin), carbacephem (such as loracarbef), carbapenems (such as imipenem), cephalosporins (such as cefadroxil, cefdinir or cefprozil), glycopeptides (such as vancomycin)macrolides (such as azithromycin or clarithromycin), penicillins (such as a amoxicillin, penicillin or cloxacillin), quinolones (such as ciprofloxacin, levofloxacin or ofloxacin), sulfonamides (such as sufacetamide) or tetracyclines (such as vibramayin or tetracycline) or any other suitable antibiotic.

The surgical intervention may be any surgical intervention or operation on the patient's body. The surgical intervention may be an elective surgery intervention or an emergency surgery intervention. Examples of such elective surgical procedures are surgery of the upper or lower gastrointestinal tract including laparoscopic procedures, open heart surgery with or without heart/lung machine, nose and throat surgery, vascular surgery, neurological (brain) surgery, transplantations (liver, heart, lung, kidney, intestinal), surgeries on the liver and caesarean sections. Examples of emergency procedures are trauma surgery in general and surgical procedures to address septic foci.

However, a preferred surgical intervention includes ischemia and/or reperfusion of at least one organ. Examples of those surgical interventions include e.g. transplantation of the liver, heart, kidney, open heart surgery etc.

The surgery intervention may be a type B and/or a type C operation. The type of surgery can be classified as described on page 208 of Dindo et al. Anals of Surgery, Vol. 240. No. 2, August 2004, pages 205 to 213. Operation type A includes surgical procedures without opening of the abdominal cavity, such as hernia repair, soft tissue surgery, thyroid surgery and excision of lymph nodes. Operation type B includes abdominal procedures except liver surgery and major surgery in the retroperitoneum, such as stomach, small bowel and colon surgery, splenectomy and cholecystectomy. Operation type C includes liver surgery, operations on the esophagus, pancreas, rectum, and retroperitoneum. The surgical interventions are preferably selected from the operations type B and/or C, in particular wherein the B type and/or C type operation is selected from the group consisting of stomach surgery, small bowel surgery, colon surgery, bowel transplantation, splenectomy, cholecystectomy, liver surgery, liver transplantation, an operation of the esophagus, an operation of the pancreas, an operation of the rectum, and an operation of the retroperitoneum, more particularly wherein the surgical intervention is selected from the group consisting of a renal surgery, kidney transplantation, lung surgery, lung transplantation, cardiac surgery, heart transplantation, neurosurgery, an urological procedure, a gynecological operation and a caesarian section.

The surgical intervention is preferably carried out on an animal, such as a mammal or preferably a human being in need of the intervention. Examples of patients being in need of such a surgical intervention are patients which are suffering from neoplasms, such as in the esophagus, pancreas, stomach, upper and lower intestine, colon and/or rectum.

As detailed above, the medicament of the invention is particularly suited to treat or preferably to prevent an infection to occur 3 to 9 days after surgical intervention, especially 4 to 7 days after surgical intervention.

In accordance with the present invention the medicament may be used in order to treat or preferably prevent any type of infection. The infection may be caused by a bacterium, virus, fungus, prion, viroid or protozoan parasite, preferably a bacterium or a virus, most preferably a bacterium. Symptoms of infection and the infectious disease result from the interplay between those few pathogens and the defenses of the hosts they infect. The appearance and severity of disease resulting from any pathogen depends upon the ability of that pathogen to damage the host as well as the ability of the host to resist the pathogen. The host's response to infection is inflammation.

Examples of viral infectious diseases are common cold, hepatitis, influenza (flu) and viral pneumonia.

Examples of bacterial infectious diseases are staphylococcal infection such as mrsa infection and urinary tract infections.

Examples of fungal infectious diseases are aspergillosis, blastomycosis, candidiasis, coccidioidomycosis and cryptococcosis.

In a more preferred embodiment of the invention the infection is selected from the group consisting of pneumonia, wound infection, wound dehiscence, intra-abdominal abscess, urinary tract infection and sepsis.

In another preferred embodiment of the invention the medicament of the invention may additionally be used for the prevention or treatment of a post-operative non-infectious complication.

In a further preferred embodiment the infection and optionally the post-operative non-infectious complication may independently be selected from the complications of Grade II and Grade III. Complications of Grade II and Grade III may be as defined in Table 1 on page 206 with clinical examples in Table 2 on page 207 of Dindo et al. Annals of Surgery, Vol. 240. No. 2, August 2004, pages 205 to 213.

Grade II complications are preferably selected from the group of complications requiring pharmacological treatment with drugs, different from antiemetics, antipyretics, analgetics, diuretics, and electrolytes, blood transfusions and total parenteral nutrition, which are usually used to treat complications of Grade I as defined in Dindo et al. Annals of Surgery, Vol. 240. No. 2, August 2004, pages 205 to 213. Examples for Grade II complications can be selected from cardiac complications, such as tachyarrythmia requiring beta-receptor antagonist for heart rate control, respiratory complications, such as pneumonia treated with antibotics (e.g. on the ward), neurological complications, such as TIA (transient ischemic attack) requiring treatment with anticoagulants, gastroinstestinal complications, such as infectious diarrhea requiring antibotics, and other complications such as any complication with requires a treatment with antibiotics.

Grade III complications are preferably selected from complications which require surgical, endoscopic or radiological interventions. Grade III complications can further be subclassified in two groups, Grade IIIa and Grade IIIb. Grade IIIa require interventions not carried out under general anesthesia and Grade IIIb require interventions performed under general anesthesia. Examples for Grade IIIa complications are cardiac complications, such as bradyarrhythmia requiring pacemaker implantation in local anesthesia, neurological complication, such as ischemic stroke/brain hemorrhage, gastrointestinal complication, such as bilioma after liver resection requiring percutaneous drainage, renal complications such as stenosis of the ureter after kidney transplanation treated with stenting, and other complications, such as closure of dehiscent noninfected wound under local anesthesia. Examples for Grade IIIb complications are cardiac complications, such as cardiac temponade after thoracic surgery requiring surgical closure, neurological complications, such as ischemic stroke/brain hemorrhage, gastrointestinal complications, such as anastomotic leakage after descendorectostomy requiring relaparotomy, renal complications, such as stenosis of the ureter after kidney transplantation treated by surgery, and other complications, such as wound infection leading to low eventration of small bowel.

In a particular preferred embodiment, the surgical intervention is selected from the group of type B and/or C operations, most preferred a surgical procedures resulting in ischemia/reperfusion of at least one organ, and the postoperative complications are selected from Group II and/or III, most preferred those which require treatment with antibiotics (e.g. pneumonia, wound infection, peritonitis, urinary tract infection or sepsis) or require surgical, endoscopic or radiological interventions with or without general anesthesia.

Still more preferably the post-operative non-infectious complication is anastomotic insufficiency and/or bilioma, most preferably an anastomotic leak. Therefore, in this embodiment the (prophylactic) treatment according to the invention reduces the rates of occurrence of these infections and of anastomotic leaks to a considerable degree and ameliorates the severity of such infections in case they still occur.

Another subject of the present invention relates to the use of a medicament comprising for the manufacture of a medicament for prevention and/or treatment of an infection following surgical intervention and optionally a post-operative non-infectious complication, wherein the above preferred embodiments and alternatives apply accordingly.

Still another subject of the invention relates to a method of preventing and/or treating an infection following surgical intervention and optionally a post-operative non-infectious complication, the method comprising administering to a patient a therapeutically effective amount of Danshen. The above preferred embodiments and alternatives may be applied accordingly.

The invention is further illustrated by the following figures and examples which are not intended in any way to limit the scope of the claimed invention.

### FIGURES

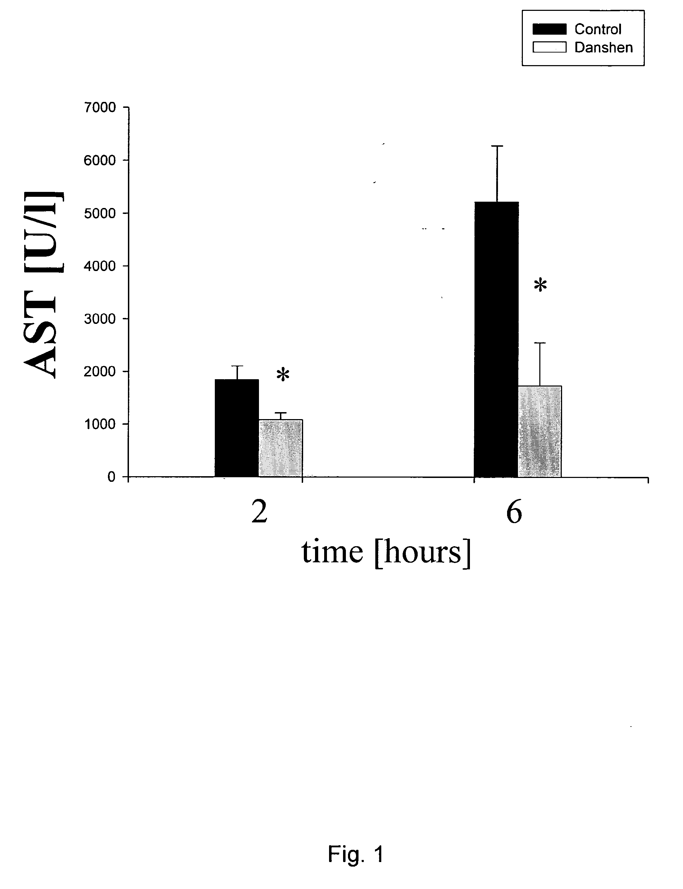

**Fig. 1** shows asparagine transferase (AST) activity after 90 min warm ischemia. Danshen was preoperatively administered in concentrations of 0 (control) or 10 g/kg body weight to rats. Asparagine transferase activity was measured after 2 and 6 h following warm ischemia for 90 min of the liver involving ischemia/reperfusion injury to the liver tissue. (*, p<0.05; Danshen compared with corresponding controls)

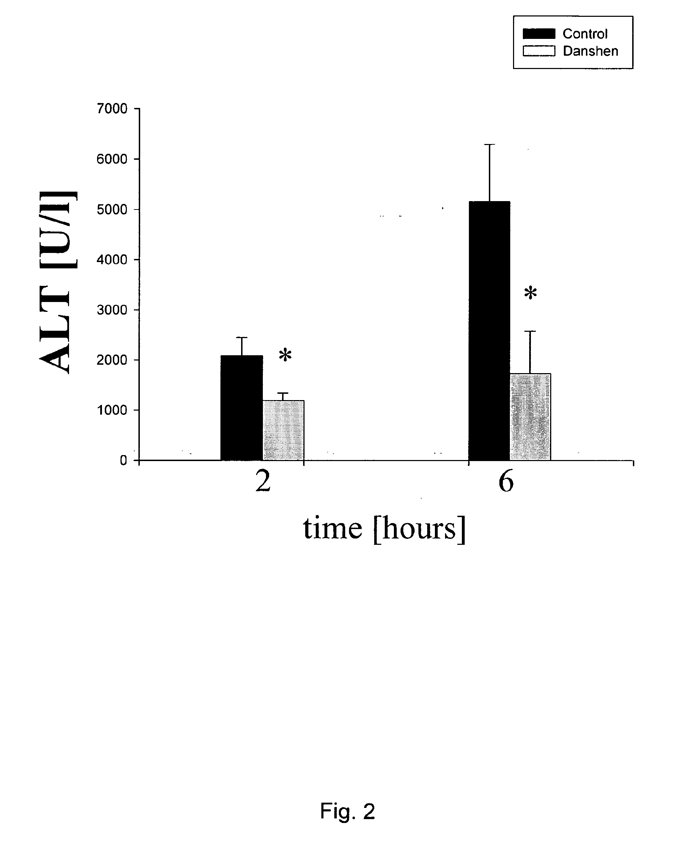

**Fig. 2** shows alanine transferase (ALT) activity after 90 min warm ischemia. Danshen was preoperatively administered in concentrations of 0 (control) or 10 g/kg body weight to rats. Asparagine transferase activity was measured after 2 and 6 h following warm ischemia for 90 min of the liver involving ischemia/reperfusion injury to the liver tissue. (*, p<0.05; Danshen compared with corresponding controls)

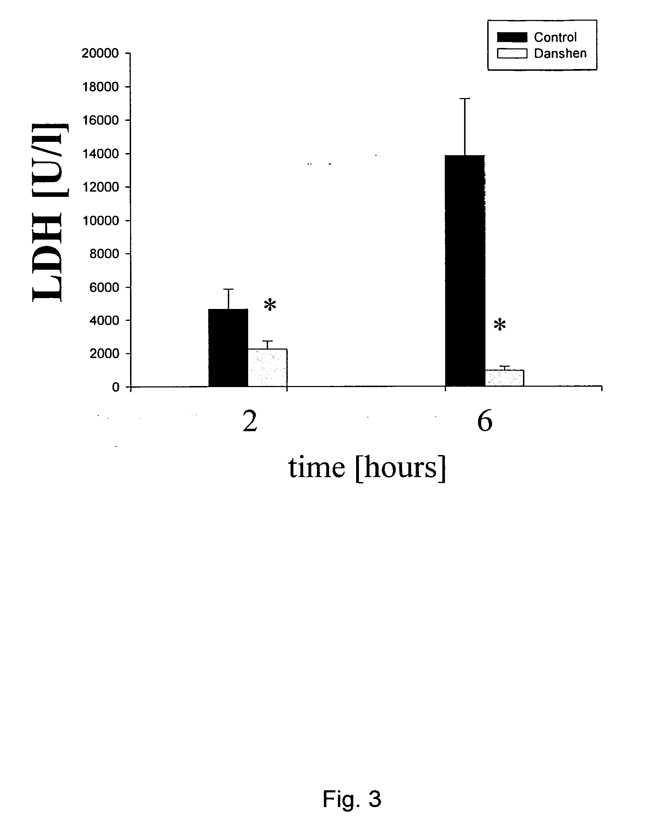

**Fig. 3** shows lactate dehydrogenase (LDH) activity after 90 min warm ischemia. Danshen was preoperatively administered in concentrations of 0 (control) or 10 g/kg body weight to rats. Asparagine transferase activity was measured after 2 and 6 h following warm ischemia for 90 min of the liver involving ischemia/reperfusion injury to the liver tissue. (*, p<0.05; Danshen compared with corresponding controls)

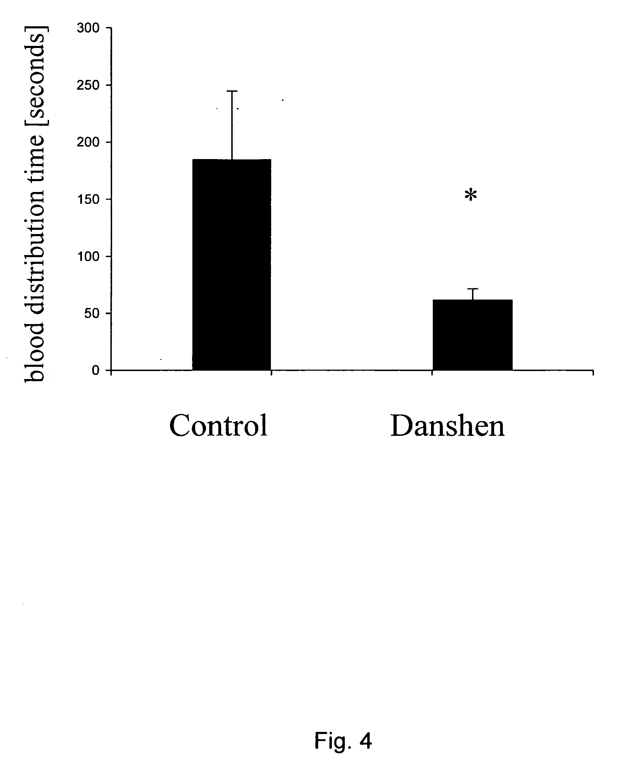

**Fig. 4** shows the liver perfusion time after transplantation of Danshen-treated donor organs. Danshen was preoperatively administered in concentrations of 0 (control) or 10 g/kg body weight to rats. Liver perfusion time after transplantation of Danshen-treated and control donor organs was determined. (*, p<0.05; Danshen compared with corresponding control)

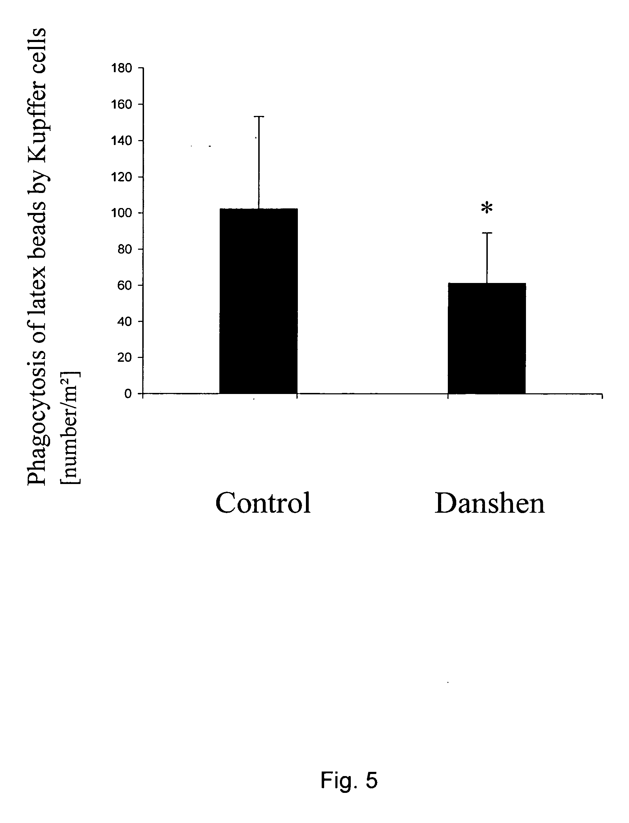

**Fig. 5** shows phagocytosis by Kupffer cells after transplantation of Danshen treated donor organs. Danshen was preoperatively administered in concentrations of 0 (control) or 10 g/kg body weight to rats. Phagocytosis of latex beads by Kupffer cells after transplantation of Danshen-treated and control donor organs was determined. (*, p<0.05; Danshen compared with corresponding control)

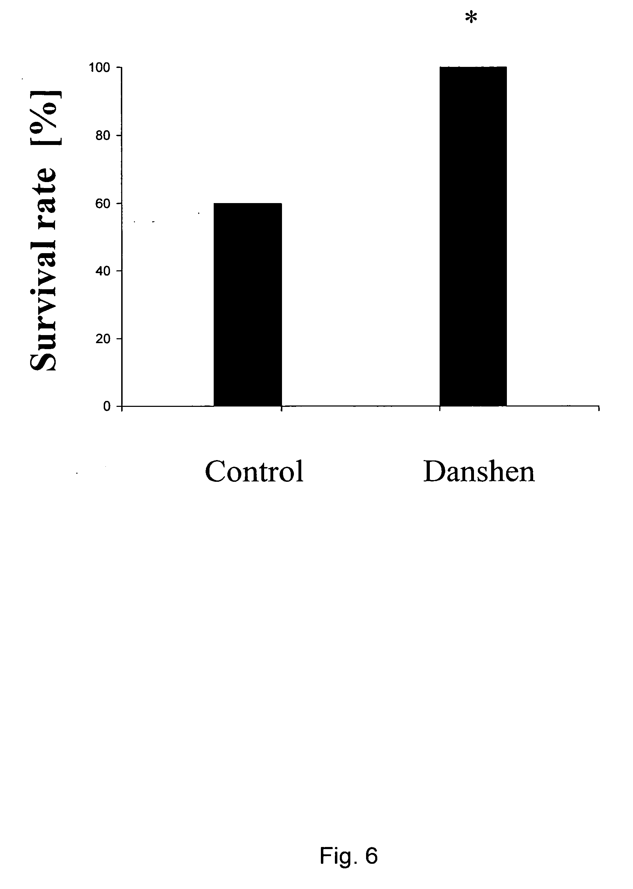

**Fig. 6** shows survival rate of grafts after transplantation of Danshen treated donor organs. Danshen was preoperatively administered in concentrations of 0 (control) or 10 g/kg body weight to rats. Survival rate of grafts after transplantation of Danshen-treated and control donor organs was determined. (*, p<0.05; Danshen compared with corresponding control)

### EXAMPLES

### Test Procedure for Example 1

Ischemia and subsequent reperfusion of organs and tissues induced by clamping of blood vessels to prevent excessive bleeding are unavoidable during surgical procedures. Such a standard ischemia/reperfusion situation can be simulated by warm ischemia of the liver over a 90 min time period in a clinically relevant rat surgical model.

In this animal model, female Sprague Dawley rats (210 - 240 g) undergo a median laparotomy followed by clamping of the left lateral liver lobe for 90 min resulting in a hypoxia in about 30 % of the entire organ. After reperfusion liver transaminases (asparagine transferase and alanine transferase) and lactate dehydrogenase were investigated.

In this rat model the operative trauma of performing a laparotomy introduces a first hypoxia in the liver. The subsequent partial clamping of the blood supply to the liver results in additional ischemia. During the subsequent reperfusion the actual free radical-induced damage to the liver tissue occurs. This actual damage to many cells of the liver tissue in regions of the remaining parts of the organ is now determined by the release of enzymes such as liver transaminases and lactate dehydrogenase after various time points following ischemia/reperfusion.

Preoperative application of Danshen by single infusion of a Danshen-containing Ringer's solution into the vena cava 10 min before the surgery results in amelioration of the massive increase in the above mentioned enzyme activities, observed in the control animals (infused with a Ringer's control solution not containing Danshen).

### Example 1

Two groups of female Spargue Dawley rats (210 - 240 g), 8 animals per group, were subjected to laparotomy followed by clamping of the left lateral liver lobe for 90 min which resulted in hypoxia of about 30 % of the whole liver. After reperfusion liver transaminases (AST and ALT) and lactate dehydrogenase (LDH) were investigated.

Danshen was administered at a concentration of 0 g/kg body weight or 10 g/kg body weight by infusion of controls (Ringer's; 0 mg/kg body weight Danshen) or a Danshen-containing Ringer's solution (1.5 g Danshen/ml, Shanghai Tongyoung Medical Co. Ltd., Z20027936, Nr. 030214, People's Republic of China) into the vena cava of 8 rats of each of the two groups 10 min before the surgery. Danshen application resulted in amelioration of the massive increase in the above mentioned enzyme activities, observed in the 8 control animals after reperfusion at 2 and 6 h. The results are shown in Figs. 1 to 3. These results indicated a reduced damage to liver tissue as a consequence of the preoperative administration of Danshen.

### Example 2

Female Spargue Dawley rats (210 - 240 g) were used as both organ donors and recipients in liver transplantation experiments. Donors were divided into two groups, 10 animals per group, in a randomized blinded fashion. Control donors were given a single infusion (1.5 ml Ringer's solution via the inferior vena cava), donors of the treatment group received 1.5 ml Danshen-containing Ringer's solution (1.5 g Danshen/ml, Shanghai Tongyoung Medical Co. Ltd., Z20027936, Nr. 030214, People's Republic of China) 10 min before surgery. Donors are essentially treated with Danshen as described in Example 1 (including test procedure). Liver transplantation was carried out according to standard procedures.

Following explantation liver grafts were stored for one hour in Ringer's solution at 4°C before standardized liver transplantation was performed. The time for complete and homogeneous distribution of blood into the graft was recorded. Immediately after complete reperfusion intravital microscopy was performed to assess intrahepatic microcirculation, leukocyte-endothelial interaction and phagocytosis of Kupffer cells. Survival was determined after an observation period of one week. Survival for more than one week was defined as permanent. The results are shown in Figs. 4 to 6.

Danshen application to donors resulted in a more rapid reperfusion of the liver graft in recipients following transplantation as can be seen in Fig. 4. The time for the liver to attain a uniform red color from inflow of blood after removing the vascular clamps was recorded. Whereas in controls 185±60 seconds were required for the blood to distribute homogeneously and completely in transplanted lievers from Danshen treated donors only 61±9 seconds were needed to achieve uniform reperfusion.

In vivo microscopy showed leukocyte accumulation within sinusoids in all subacinar zones and in postsinusoidal venules in both experimental groups (Table 1). The administration of Danshen prior to organ harvest, significantly reduced the number of permanent adherent leukocytes in both sinusoids and venules from 107±8 /mm² and 650±41 /mm² respectively to about 60% of controls immediately after reperfusion (Table 1).

Also a substantial sinusoidal perfusion disturbance was observed in both groups immediately after reperfusion. A significant increase in sinusoidal perfusion was observed in Danshen treated grafts as against control grafts (Table 1). In parallel, Danshen application lead to a 60 % decrease in non-perfused sinusoids within perfused acini compared to controls (Table 1).

Velocity of RBCs, diameter of the sinusoids and blood flow were similar in both groups in different zones immediately after reperfusion. However, while blood flow and RBC velocity decreased in all sinusoidal of controls, Danshen markedly increased the blood flow by 40 % and velocity of RBCs by 20 % in different sinusoidal zones (Table 1). In addition, Danshen significantly expanded the diameter of capillary vessel in all sinusoidal zones (Table 1).

To asses the activation of Kupffer cells, the number of latex beads phagocytozed by Kupffer cells were compared between groups. Danshen significantly reduced phagocytosis after transplantation to about 40% of mean control values (Fig. 5).

Similarly, a significant increase in survival after 7 days was observed as an effect of the donor treatment with Danshen (Fig. 6). Sixty percent of grafts from controls, stored for one hour in cold Ringer's solution, survived for one week after liver transplantation. 1.5 ml Danshen solution, given as an infusion to donors before harvest, improved the survival rate to 100 % (p<0.05). Percent survival did not change within the groups during the second week of observation.

These effects demonstrate the benefits of preoperative application of Danshen before major surgery in preventing injury through procedure related ischemia/reperfusion situations and indicate the potential of preventing/ameliorating the occurrence of postoperative infectious and non-infectious complications.

**Table1**

| Parameter | | Control | Danshen |
|---|---|---|---|
| **Sinusoidal perfusion** | | | |
| | Perfusion Index | 0.94±0.02 | 0.98±0.06* |
| | Non-perfusion sinusoidal | 4.7±1.5 | 1.9±0.06* |
| | | | |

| **Sinusoidal Speed (mm/s)** | | | |
|---|---|---|---|
| | Non-perfusion sinusoidal | 0.21±0.01 | 0.31±0.02* |
| | Zone2 (Mid-zone) | 0.29±0.01 | 0.40±0.02* |
| | Zone3 (Peri-central) | 0.43±0.02 | 0.57±0.03* |
| | Post-venule Speed (mm/s) | 0.87±0.06 | 0.95±0.06 |
| | | | |

| **Sinusoidal Diameter (um)** | | | |
|---|---|---|---|
| | Zone1 (Peri-portal) | 8.35±0.11 | 10.62±0.13* |
| | Zone2 (Mid-zone) | 9.85±0.11 | 13.46±0.16* |
| | Zone3 (Peri-central) | 10.88±0.12 | 14.66±0.18* |
| | Post-venule Diameter (um) | 26.89±0.53 | 27.85±0.5 |
| | | | |

| **Sinusoidal Flow (mm3/s ×10⁻⁶)** | | | |
|---|---|---|---|
| | Zone1 (Peri-portal) | 11.52±1.34 | 28.86±5.38* |
| | Zone2 (Mid-zone) | 22.80±2.57 | 59.05±8.32* |
| | Zone3 (Peri-central) | 40.65±4.82 | 100.74±13.99* |
| | Post-venule Flow (mm³/s×10⁻⁶) | 598.58±136.7 | 629.24±72.24 |
| | | | |

| **Adherence of leukocytes** | | | |
|---|---|---|---|
| | Rollers in venules [n/mm²] | 185.8±42.4 | 22.5±13.7* |
| | Sticks in venules [n/mm²] | 650.2±82.7 | 108.4±20.2* |
| | Sticks in sinusoidal [n/mm²] | 107.1±7.6 | 15.0±3.4* |

Before organ harvesting, some donor rats were infused with Danshen 1.5 ml as described above. Immediately after transplantation, the hepatic microcirculation was observed as described above. Leukocyte-endothelium interaction has been monitored to study temporal (roller) and permanent leukocyte adhesion (sticker) to endothelial lining cells in both sinusoids and venules. Further, sinusoidal perfusion, velocity of erythrocyte, blood flow and diameter have been assessed. Values are mean ± SEM (p<0.05 by two-way ANOVA with Student-Newman-Keuls post-hoc test, n=6-8). *, p<0.05 for comparison to corresponding controls.

## Claims

1. A medicament comprising Danshen for use in prevention and/or treatment of an infection following surgical intervention.

2. The medicament according to claim 1, wherein for use in prevention of an infection following surgical intervention.

3. The medicament according to claim 1 or 2, wherein the medicament is to be administered prior to the surgical intervention, preferably wherein the medicament is to be administered at most approximately 24 hours prior to the surgical intervention, preferably at most approximately 12 hours prior to the surgical intervention, more preferably at most approximately 6 hours prior to the surgical intervention, even more preferably at most approximately 3 hours prior to the surgical intervention, still more preferably at most approximately 2 hours prior to the surgical intervention, most preferably at most approximately 1 hour prior to the surgical intervention.

4. The medicament according to any of claims 1 to 3, wherein the medicament is to be administered in a dose of from 0.1 to 5000 mg/kg body weight, preferably 1 to 2000 mg/kg body weight, more preferably 5 to 1000 mg/kg body weight and most preferably 50 to 500 mg/kg body weight.

5. The medicament according to any of claims 1 to 4, wherein the medicament is to be administered at most three times, preferably at most twice or once before or during the surgical intervention.

6. The medicament according to any of claims 1 to 5, wherein the medicament is in a form intended for oral administration, preferably a drinking solution, tablet, a soft gelatin capsule or a hard gelatin capsule, or in a form intended for parenteral administration, preferably for intravenous or intra-arterial application.

7. The medicament according to any of claims 1 to 6, wherein the Danshen is used as the sole therapeutically active agent.

8. The medicament according to any of claims 1 to 7, wherein the medicament is used in combination with another infection prevention and/or treatment agent, preferably in combination with an antibiotic.

9. The medicament according to any of claims 1 to 8, wherein the surgery intervention is an elective or an emergency surgery intervention.

10. The medicament according to any according to any of claims 1 to 9, wherein the surgical intervention includes ischemia and/or reperfusion of at least one organ.

11. The medicament according to any of claims 1 to 10, wherein the surgical intervention is a type B and/or a type C operation.

12. The medicament according to claim 11, wherein the B type and/or C type operation is selected from the group consisting of stomach surgery, small bowel surgery, colon surgery, bowel transplantation, splenectomy, cholecystectomy, liver surgery, liver transplantation, an operation of the esophagus, an operation of the pancreas, an operation of the rectum, and an operation of the retroperitoneum.

13. The medicament according to claim 12, wherein the surgical intervention is selected from the group consisting of a renal surgery, kidney transplantation, lung surgery, lung transplantation, cardiac surgery, heart transplantation, neurosurgery, an urological procedure, a gynecological operation and a caesarian section.

14. The medicament according to any of claims 1 to 13, wherein the surgical intervention was performed on a mammal, preferably human.

15. The medicament according to any of claims 1 to 14, wherein the infection to be treated or preferably to be prevented is to occur 3 to 9 days after the surgical intervention, especially 4 to 7 days after the surgical intervention.

16. The medicament according to any of claims 1 to 15, wherein the infection is caused by bacterium, virus, fungus, prion, viroid or protozoan parasite, preferably a bacterium or a virus, most preferably a bacterium.

17. The medicament according to any of claims 1 to 16, wherein the infection is selected from the group consisting of pneumonia, wound infection, wound dehiscence, intra-abdominal abscess, urinary tract infection and sepsis.

18. The medicament according to claims 1 to 17, wherein the medicament is additionally used for the prevention or treatment of a post-operative non-infectious complication.

19. The medicament according to claims 1 to 18, wherein the infection and optionally the post-operative non-infectious complication are independently selected from the complications of Grade II and/or Grade III.

20. The medicament according to claim 18 or 19, wherein the post-operative non-infectious complication is anastomotic insufficiency and/or bilioma.

21. Use of Danshen for the manufacture of a medicament as defined in any of claims 2 to 20 for prevention and/or treatment of an infection following surgical intervention and optionally a post-operative non-infectious complication.
